# EUROPÄISCHE PATENTSCHRIFT

(11) **EP 2 802 372 B1**
(45) Veröffentlichungstag und Bekanntmachung des Hinweises auf die Patenterteilung: **21.12.2016**
(21) Anmeldenummer: 13700088.1
(22) Anmeldetag: 09.01.2013
(51) Int. Cl.: A61M 39/02, A61M 5/32

(54) **PORTKANÜLE ZUM PUNKTIEREN VON PORTKATHETERN**
PORT CANNULA FOR PUNCTURING PORT CATHETERS
CANULE POUR CHAMBRE IMPLANTABLE DESTINÉE AU POINTAGE DE CATHÉTERS POUR CHAMBRE IMPLANTABLE

(30) Priorität: 09.01.2012 EP 12150516; 09.01.2012 US 201261584387 P
(43) Veröffentlichungstag der Anmeldung: 19.11.2014
(73) Patentinhaber: Fresenius Kabi Deutschland GmbH, 61352 Bad Homburg (DE)
(72) Erfinder: PAPIOREK, Martina, 65597 Hünfelden (DE)
(74) Vertreter: Kusche, Robert
(86) Internationale Anmeldenummer: PCT/EP2013/050252
(87) Internationale Veröffentlichungsnummer: WO 2013/104642

(56) Entgegenhaltungen:
- EP-A1- 0 495 214
- WO-A2-01/34239
- DE-U1-202006 010 322
- US-A- 2 717 599

## Beschreibung

### Beschreibung der Erfindung

Die vorliegende Erfindung bezieht sich auf eine Portkanüle zum Punktieren von implantierbaren bzw. implantierten Portkathetern.

### Hintergrund der Erfindung

Bekannte Kanülen (oder kurz Punktionskanülen) zum Punktieren oder Einsteichen weisen einen mehr oder weniger stark geneigten keilförmigen Anschliff am einem Endabschnitt der Kanüle auf. Die hintere Kante des keilförmigen Anschliffs bildet dabei eine scharfe Schneide. Eine derartige Schneide führt bei der Punktion dazu, dass Bestandteile aus dem zu durchstechenden Material ausgestanzt werden. Dies ist zum Beispiel bei einer Gewebepunktion aufgrund der Traumatisierung unerwünscht. Weiterhin erweist sich der Ausstanzeffekt als äußerst unerwünscht, wenn derartige Kanülen zum Punktieren von implantierbaren Port-Kathetern eingesetzt werden. Solche Port-Katheter bestehen in der Regel wenigstens aus einer implantierten Kapsel, die einen Hohlraum zur Entnahme und/oder zur Applikation einer Flüssigkeit wie Blut, Blutkomponenten oder eines Medikaments aufweist. Die Kapsel ist mit einem Katheter verbunden, der in ein Gefäß oder eine sonstigen Wirkort mündet. Die zur Haut des Patienten hingerichtete Wand der implantierten Kapsel wird durch eine punktierbare Elastomer-Membran bereitgestellt, die mit der Kanüle durch die Haut durchstochen wird. Das Einstechen einer Kanüle in die Membran eines Port-Katheters verursacht Undichtigkeiten dadurch, dass die hintere Kante des Anschliffs Elastomer-Material aus der Membran ausstanzt. Dadurch entstehen Löcher, die sich nicht mehr über die Rückstellkraft des Membranmaterials von selbst schließen können. Dies führt dazu, dass die implantierte Kapsel schon nach wenigen Einstichen Leckagen aufweist. Weiterhin können ausgestanzte Elastomer-Partikel in den Patienten geschwemmt werden oder den Katheter verstopfen.

In dem europäischen Patent EP 0 495 214 B1 ist eine Portkanüle beschrieben, mittels welcher ein Ausstanzen wirksam reduziert werden kann. Der Inhalt dieses Dokuments wird durch Bezugnahme vollumfänglich in die vorliegende Patentanmeldung inkorporiert. Der dort beschriebenen Kanüle liegt der Ansatz zugrunde, dass die durch die hintere Kante gebildete Schneide durch die Kanülenspitze im Wesentlichen verdeckt ist und dadurch nicht mehr schneiden kann. Das wird im Wesentlichen dadurch erreicht, dass das Kanülenrohr zum einstechenden Teil hin seitlich abgeknickt und im weiteren Verlauf hakenförmig ausgebildet ist und eine in die konkave Ausformung des hakenförmig verlaufenden Rohres tangential eingeschliffene Lumenöffnung aufweist, die an ihrer Hinterkante eine, durch die Anformung des Rohres bedingte, stark einwärts gerichtete Schneide ausbildet. Die an eine Portkanüle gestellten Anforderungen werden durch die in der EP 0 495 214 B1 beschriebene Portkanüle im Wesentlichen erfüllt.

### Allgemeine Beschreibung der Erfindung

Vor dem vorstehend geschilderten Hintergrund liegt der vorliegenden Erfindung die Aufgabe zugrunde, eine Kanüle zum Punktieren von Port-Kathetern bereitzustellen, wobei das Punktionsverhalten der Kanüle noch weiter verbessert werden soll.

Gelöst wird diese Aufgabe bereits durch die Kanüle gemäß dem unabhängigen Patentanspruch 1. Vorteilhafte Ausführungsformen der erfindungsgemäßen Kanüle sind Gegenstand der abhängigen Unteransprüche.

Die Erfinder haben erkannt, dass die Leckagen in der Membran eines Port oder Portkatheters nicht nur aus einem Ausstanzen von Membranmaterial beim Einführen der Kanüle resultieren können. Vielmehr kann die Kanüle auch beim Rausziehen Membranmaterial mit sich reißen. Insbesondere wird dies dadurch verursacht, dass die Kanülenspitze nach dem Durchdringen der Membran auf den Portboden auftreffen und verbogen werden kann. Dadurch bildet sich eine Art Angelhaken oder Haken an der Kanülenspitze, der beim Herausziehen Material aus der Membran sozusagen "herauskratzen" kann. Diese Hakenbildung wird insbesondere begünstigt durch eine unsachgemäße Benutzung der Kanüle, wenn zum Beispiel die Kanüle mit zu viel Kraft und/oder über einen falschen Winkel in den Portkatheter eingeführt wird. Hierbei erweist sich eine scharfe Spitze als nachteilig, da diese eine geringere Stabilität besitzt und sich leichter zu einem Haken verformen kann. Andererseits erweist sich eine scharfe Spitze als vorteilhaft, da diese beim Einführen einen geringeren Kraftaufwand erfordert. Je geringer die erforderliche Einstechkraft ist, desto weniger schmerzhaft erweist sich die Punktierung für den Patienten.

Allgemein sieht die Erfindung vor, den Anschliff der Kanüle, insbesondere den Grundschliff und den Facettenschliff, so zu gestalten bzw. aufeinander abzustimmen, dass zum einen eine Hakenbildung möglichst vermieden oder zumindest reduziert wird und zum anderen die Schmerzen für den Patienten beim Punktieren in einem akzeptablen Bereich gehalten werden.

Im Detail wird die vorliegende Erfindung beschrieben durch eine Kanüle zum Punktieren eines implantierbaren Ports. Die Kanüle oder Portkanüle ist aufgebaut wenigstens aus
einem Kanülenrohr mit einem Anschliff, welcher sich zumindest aus einem gegenüber einer Mittelachse des Kanülenrohrs um einen Grundschliffwinkel (β) geneigten Grundschliff und zwei im Winkel (γ) zueinander stehenden Facettenschliffen zusammensetzt, und einer durch den Anschliff bereitgestellten Kanülenspitze, welche über eine erste Biegung in Richtung auf die Mittelachse der Kanülenrohrs gerichtet ist, dadurch gekennzeichnet, dass
der Grundschliffwinkel (β) in einem Bereich von β = 13° bis 22°, bevorzugt von β = 14° bis 19°, besonders bevorzugt von β = 15° bis 17°, liegt und
der Winkel (γ) zwischen den zwei Facettenschliffen (12) in einem Bereich von γ = 90° bis 120°, bevorzugt von γ = 100° bis 115°, besonders bevorzugt von γ = 105° bis 110°, liegt.

Der Grundschliff und der Facettenschliff sind so aufeinander abgestimmt, dass beim Punktieren zum einen ein Verbiegen der Kanüle und somit eine Hakenbildung der Kanüle zumindest reduziert werden und zum anderen die Schmerzen für den Patienten in einem akzeptablen Bereich gehalten werden.

Die Kanülenspitze kann sich hierbei auch über die Mittelachse des Kanülenrohrs hinaus erstrecken. Die Mittelachse des Kanülenrohrs als Bezugsgröße bezieht sich immer auf den nicht gebogenen bzw. geraden Abschnitt der Kanüle. Die Kanülenspitze wird nicht nur durch den vordersten Punkt bzw. den Einstechpunkt der Kanüle bereitgestellt. Vielmehr wird die Kanülenspitze durch den Bereich des Anschliffs bereitgestellt, der den eigentlichen Schneidprozess beim Einstechen durch die Haut eines Patienten und die Membran eines Ports ermöglicht. Insbesondere wird die Kanülenspitze durch den Bereich der Kanüle bereitgestellt, an denen die Facettenschliffe angeordnet sind. Alternativ kann die Kanüle aufgebaut sein wenigstens aus einem Kanülenrohr mit einem Anschliff, der sich zumindest aus einem gegenüber einer Mittelachse des Kanülenrohrs um einen Grundschliffwinkel (β) geneigten Grundschliff und zwei im Winkel (γ) zueinander stehenden Facettenschliffen zusammensetzt, und einer durch den Anschliff bereitgestellten Kanülenspitze, welche über eine erste Biegung in Richtung auf die Mittelachse der Kanülenrohrs gerichtet ist, wobei der Grundschliffwinkel (β) in einem Bereich von β = 13° bis 22°, bevorzugt von β = 14° bis 19°, besonders bevorzugt von β = 15° bis 17°, liegt und/oder der Winkel (γ) zwischen den zwei Facettenschliffen (12) in einem Bereich von γ = 90° bis 120°, bevorzugt von γ = 100° bis 115°, besonders bevorzugt von γ = 105° bis 110°, liegt.

In einer ersten Ausführungsform der Erfindung weist der Grundschliff einen Anschliffbereich der Länge a auf. Wenigstens einer der zwei Facettenschliffe besitzt eine Länge c von c < 1/3 a. Vorzugsweise besitzen beide Facettenschliffe eine Länge c von c < 1/3 a. Dadurch wird das Ausstanzen noch weiter verringert.

In einer Aufsicht auf die Vorderseite des Kanülenrohrs entlang der Mittelachse der Kanüle ist ein Lumen des Kanülenrohrs durch die Kanülenspitze zumindest abschnittweise verdeckt. Vorzugsweise wird dabei eine durch den Grundschliff bereitgestellte Hinterkante durch die Kanülenspitze zumindest abschnittsweise, insbesondere vollständig, verdeckt. Dadurch kann das Ausstanzen noch weiter vermindert werden. In einer bevorzugten Ausführungsform liegt ein Einstechbereich oder einer vorderster Bereich der Kanülenspitze in einem Bereich g von 0,5 mm oberhalb und 0,2 mm unterhalb, bevorzugt 0,2 mm oberhalb und 0,1 mm unterhalb, der Mittelache des Kanülenrohrs. Vorzugsweise liegt die Kanülenspitze in einem Bereich oberhalb der Mittelachse des Kanülenrohrs.

In einer weiteren Ausführungsform der Erfindung wird die erste Biegung der Kanülenspitze durch einen, vorzugsweise einzelnen, ersten Biegeradius (r) bereitgestellt. Der erste Biegeradius liegt insbesondere in einem Bereich von r = 5 mm bis 10 mm, bevorzugt von 7 mm bis 9 mm. Die erste Biegung der Kanülenspitze weist einen ersten Scheitelpunkt auf. Vorzugsweise liegt dieser erste Scheitelpunkt in einem Abstand e = 3 mm bis 5 mm, bevorzugt von 3,6 mm bis 4 mm zu einem Einstechbereich der Kanülenspitze.

Insbesondere um ein mögliches Ausstanzen noch weiter zu reduzieren, ist ein zwischen den zwei Facettenschliffen liegender Abschnitt des Grundschliffs zumindest abschnittsweise, vorzugsweise vollständig, verrundet. Im Detail sind die Kanten dieses Abschnitts verrundet. Die Verrundung erfolgt hierbei durch ein nicht-abrasives Verfahren. Mögliche Beispiele für nicht-abrasive Verfahren stellen Glasperlenstrahlen, Trockeneisstrahlen und/oder CO2-Schneestrahlen dar. Vorzugsweise sind alle Kanten der Anschliffs, mit Ausnahme des Facettenschliffe, verrundet.

Die erfindungsgemäße Kanüle weist in einer bevorzugten Ausführungsform wenigstens eine zweite Biegung auf. Ein Endabschnitt der Kanüle, an dem der Anschliff angeordnet ist, ist über eine zweite Biegung von der Mittelachse der Kanülenrohrs weg gerichtet ist. Insbesondere ist der Endabschnitt der Kanüle, an dem der Anschliff angeordnet ist, über die zweite Biegung in eine Richtung gebogen ist, welche der Richtung der ersten Biegung gegenüberliegt. Vorzugsweise liegen die Richtungen der ersten Biegung und der zweite Biegung in einem Winkel von 160° bis 200°, bevorzugt von 175° bis 185°, besonders bevorzugt von etwa 180°, zueinander. Die Benennung der Biegungen ergibt sich aus der Reihenfolge, wie die Biegungen, ausgehend von der Vorderseite bzw. dem Einstechbereich der Kanüle aus angeordnet sind. Durch die erste Biegung und die zweite Biegung wird eine Art Löffel gebildet. Daher wird eine solche Kanüle auch vereinfacht als Kanüle mit Löffelbiegung bezeichnet. Die Ausgestaltung der Kanüle mit Löffelbildung erweist sich hierbei als vorteilhaft, da die Position bzw. Ausrichtung der Kanülenspitze über die erste Biegung in Bezug auf die Mittelachse des Kanülenrohrs weniger kritisch ist.

Eine Ausgestaltung ist derart, die zweite Biegung des Endabschnitts einen zweiten Scheitelpunkt aufweist, der in einem Abstand f = 4 mm bis 10 mm, bevorzugt f = 6 mm bis 10 mm, besonders bevorzugt f = 6 mm bis 8 mm, zu einem Einstechbereich der Kanülenspitze liegt. Vorzugsweise wird der Endabschnitt der Kanüle, an dem der Anschliff angeordnet ist, in einem spitzen Winkel (α) zu der Mittelachse des Kanülenrohrs angeordnet ist. Insbesondere liegt der spitze Winkel (α) in einem Bereich von α = 1° bis 10°, bevorzugt α = 4° bis 7°.

Eine weitere Ausführungsform der Erfindung ist dadurch gekennzeichnet, dass zumindest ein Abschnitt einer Außenseite des Kanülenrohrs eine aufgeraute Oberfläche hat. Dadurch kann die Handhabung der Kanüle verbessert werden. Insbesondere kann dadurch ein sicheres Befestigen einer Zuführung (siehe nachfolgend) ermöglicht werden. Der Abschnitt mit der aufgerauten Oberfläche erstreckt sich vorzugsweise nicht vollständig über den Umfang des Kanülenrohrs. Insbesondere besitzt dieser Abschnitt eine Rauigkeit Rz, die in einem Bereich von Rz = 1 µm bis 10 µm, bevorzugt von Rz = 5 µm bis 10 µm, liegt. Vorzugsweise ist der Abschnitt der Außenseite mit der aufgerauten Oberfläche in einem Abstand von etwa 1 mm von einer Rückseite des Kanülenrohrs angeordnet und/oder besitzt der Abschnitt der Außenseite mit der aufgerauten Oberfläche eine Längserstreckung (entlang der Mittelachse) von etwa 2 mm bis 15 mm, bevorzugt von etwa 4,5 bis 6,0 mm.

Als Material für die Kanüle wird ein biokompatibles Metall verwendet. Ein bevorzugtes Material für die Kanüle stellt nichtrostender austenitischer Stahl dar. Ein Beispiel dafür ist X5CrNi18-10 (Werkstoff-Nr. 1.4301)

Weiterhin liegt im Bereich der vorliegenden Erfindung auch ein Portkanülensystem, welches eine Ausführungsform einer vorstehend beschriebenen erfindungsgemäßen Kanüle umfasst. Weiterhin umfasst das Portkanülensystem eine mit der Kanüle verbundene Zuführung zum Anschließen eines Schlauchs eines Überleitsystems. Die Zuführung ist hierbei vorzugsweise an einem dem Endabschnitt gegenüberliegenden Abschnitt des Kanülenrohrs angeordnet. Das Portkanülensystem kann zudem eine mit der Zuführung verbundene Auflage aufweisen zur Auflage und insbesondere zum Befestigen des Portkanülensystems auf der Haut eines Patienten. Ein Beispiel für die Auflage ist eine Art Pflaster. In einer Ausgestaltung ist die Zuführung an dem Abschnitt der Außenseite des Kanülenrohrs mit der aufgerauten Oberfläche angeordnet.

Die mit der erfindungsgemäßen Kanüle getesteten Portsysteme haben sich auch nach etwa 150 Punktierungen noch als funktionsfähig erwiesen. Da die Portsysteme implantiert sind, kann das medizinische Personal das Portsystem beim Punktieren nicht sehen sondern nur ertasten. Es kann somit nicht sichergestellt werden, dass das Punktieren immer im Zentrum der Membran und/oder immer vertikal erfolgt. Dies ist mit der erfindungsgemäßen Kanüle auch nicht erforderlich.

Die vorliegende Erfindung wird anhand der nachfolgenden Ausführungsbeispiele im Einzelnen erläutert. Hierzu wird auf die beigefügten Zeichnungen Bezug genommen. Die gleichen Bezugszeichen in den einzelnen Zeichnungen beziehen sich auf die gleichen Teile.
- Fig. 1: zeigt eine schematische Darstellung eines subkutan implantierten Portkatheters.
- Fig. 2.a bis c: illustrieren den Endabschnitt einer Kanüle in einer Aufsicht auf den Anschliff (Fig. 2.a) sowie in einer Seitenansicht ohne (Fig. 2.b) und mit Maßangaben (Fig. 2.c).
- Fig. 3.a bis c: illustrieren die Herstellungsschritte der erfindungsgemäßen Kanüle in einer Seitenansicht.
- Fig. 4.a und b: zeigen eine photographische Darstellung einer erfindungsgemäßen Kanüle mit (Fig. 4.a) und ohne (Fig. 4.b) Kanülenrohr.
- Fig. 5.a und b: zeigen zum Vergleich eine schematische Darstellung (Fig. 5.a) und ein Foto (Fig. 5.a) einer erfindungsgemäßen Kanüle.
- Fig. 6.a und b: zeigen eine schematische Darstellung eines Portkanülensystem ohne (Fig. 6.a) und mit Auflage Foto (Fig. 6.a).

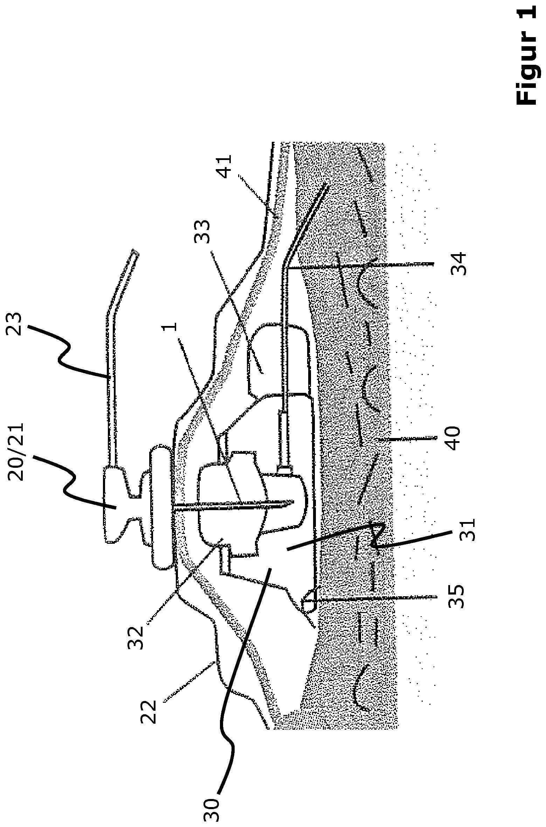

Figur 1 zeigt einen implantierten Portkatheter 30. Der Portkatheter 30 ist unter der Haut 41 implantiert und wird über Befestigungsmittel 35 mit dem umliegenden Gewebe 40 verbunden, um ein Verrutschen zu verhindern. Der Portkatheter 30 wird wenigstens durch ein Gehäuse 31, eine elastomere Membran 32 (z.B aus Silikon) und einen an dem Gehäuse 31 mittels eines Anschlusses 33 angeschlossenen Katheter 34 breitgestellt. Der Katheter 34 stellt die Verbindung des Ports 30 mit dem umliegenden Gewebe 41, beispielsweise einer Vene, her. Um einem Patienten zum Beispiel ein intravenös zu verabreichendes Medikament zuzuführen, wird die Membran 32 durch die Haut 41 hindurch mittels einer Kanüle 1 oder Portkanüle 1 durchstoßen. Die Kanüle 1 stellt die Verbindung zwischen einer in dem Gehäuse 31 ausgebildeten Infusionskammer zu einem nicht in der Figur dargestellten Infusionsbeutel bereit. Ein Portkatheter 30 kann eine zugelassene Implantationszeit von bis zu 5 Jahren besitzen. Entsprechend oft kann der Portkatheter 30 durch eine Portkanüle 1 punktiert werden. Die Portmembran 32 darf durch die Punktierung mittels der Kanüle 1 nicht wesentlich geschädigt werden. Um einen dauerhaften Betrieb eines Portkatheters 30 zu gewährleisten, können nur spezielle Portkanülen 1 verwendet werden. Die Anforderungen an solche spezielle Kanülen werden durch die erfindungsgemäße Kanüle 1 wesentlich erfüllt.

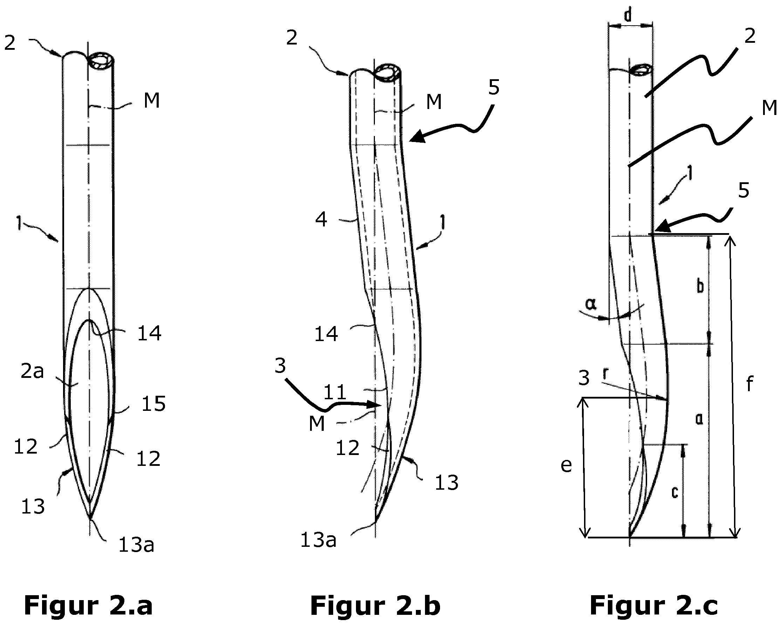

Die Figuren 2.a bis c illustrieren den Endabschnitt 4 einer Kanüle 1 in einer Aufsicht auf den Anschliff 10 (Fig. 2.a) sowie in einer Seitenansicht ohne (Fig. 2.b) und mit Maßangaben (Fig. 2.c). Die Figur 2.a zeigt eine Aufsicht auf die Oberseite des Anschliffs 10. Zu erkennen ist die durch den Grundschliff 11 erzeugte Hinterkante 14. Zumindest der obere innere Bereich der Hinterkante 14 wird wesentlich durch die Kanülenspitze 13 verdeckt, um ein Ausschneiden durch die Hinterkante 14 möglichst zu vermeiden. Der vorderste Bereich 13a der Kanülenspitze 13 stellt den Einstechbereich 13a der Kanüle 1 dar. Durch die beiden Facettenschliffe 12 erfolgt das eigentliche Schneiden durch die Haut 41 und durch die Membran 32. Hinter den beiden Facettenschliffen 12 liegt der Bereich 15 des Grundschliffs 11, der verrundet wird. Vorzugsweise sind alle Kanten der Anschliffs 10 verrundet, mit Ausnahme der Facettenschliffe 12. Diese sind nicht verrundet, da diese den Schneidvorgang durchführen. Der Einstechbereich 14 liegt in dieser Ansicht auf der Mittelachse M der Kanüle 1.

Weiterhin zeigen die Figuren 2.b und 2.c eine Seitenansicht der Kanüle 1 mit zum Teil angedeutetem Querschnitt. Aus Darstellungsgründen wurden diese Figuren aus der EP 0 495 214 B1 übernommen. Die in der vorliegenden Patentanmeldung beschriebene Kanüle 1 hat jedoch ein anderes Design.

In Figur 2.b sind zunächst die einzelnen Merkmale der Kanüle 1 angegeben. Der Endabschnitt 4, der den Anschliff 10 trägt, ist gegenüber dem Kanülenrohr 2 bzw. der Mittelachse M des Kanülenrohrs 2 über eine zweite Biegung 5 geneigt oder gebogen. Die Kanülenspitze 13 ist über oder durch eine erste Biegung 3 wieder in Richtung der Mittelachse M des geraden Kanülenrohrs 2 gerichtet oder gebogen. Der Einstechbereich 13a der Kanülenspitze 13 liegt hier auf der Mittelachse M des nicht gebogenen Kanülenrohrs 2. Der Einstechbereich 13a der Kanülenspitze 13 oder die Kanülenspitze 13 als solche kann sich in dieser Ebene auch abschnittsweise über die Mittelachse M des Rohrs 2 hinaus erstrecken.

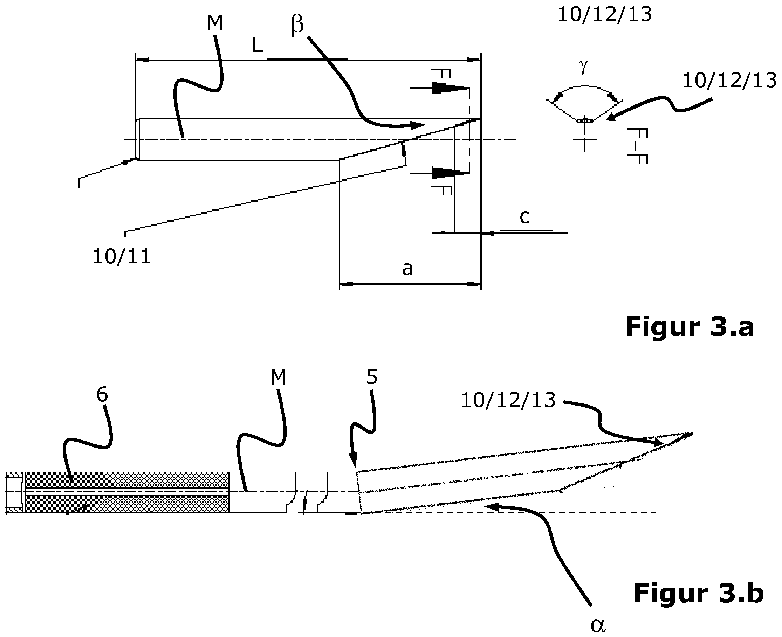

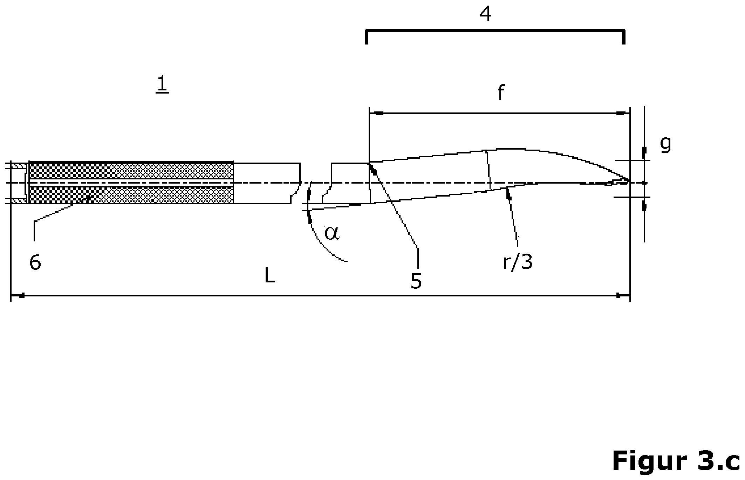

In Figur 2.c sind nun die einzelnen Längenangaben der Kanüle 1 angegeben. Die Kanüle besitzt einen Durchmesser d. Der Grundschliff 11 ist durch den Winkel β charakterisiert (siehe dazu Figur 3.a). Der Winkel β ist unabhängig von dem Durchmesser d der Kanüle 1. Für eine 19G-Nadel (Durchmesser d = 1,1 mm) hat sich eine Schlifflänge a von a = 3,2 mm bis 4,4 mm, vorzugsweise von a = 3,6 mm bis 4,0 mm, als vorteilhaft erwiesen. Für eine 20G-Nadel (Durchmesser d = 0,9 mm) hat sich eine Schlifflänge a von a = 2,4 mm bis 3,6 mm, vorzugsweise von a = 2,7 mm bis 3,1 mm, als vorteilhaft erwiesen. Für eine 22G-Nadel (Durchmesser d = 0,7 mm) hat sich eine Schlifflänge a von a = 1,4 mm bis 3,0 mm oder bis kleiner 3,0 mm, vorzugsweise von a = 1,8 mm bis 2,4 mm, als vorteilhaft erwiesen. Der Winkel α beschreibt den Winkel der zweiten Biegung 5 des Endabschnitts 4. Der Bereich, in dem der Scheitelpunkt der zweiten Biegung 5 (der zweite Scheitelpunkt) liegt, ist mit der Pfeilspitze vom Bezugszeichen 5 angedeutet. Der Endabschnitt 4 besitzt eine Länge f. Die Länge f beschreibt den Abstand zwischen dem vorderen Punkt 13a und dem zweiten Scheitelpunkt. Der Krümmungsradius r beschreibt die erste Biegung 3 der Kanülenspitze 13. Der Bereich, in welchem der Scheitelpunkt der ersten Biegung 3 (der erste Scheitelpunkt) liegt, ist mit der Pfeilspitze vom Bezugszeichen 3 angedeutet. Der Abschnitt der Kanülenspitze 13, der gebogen wird, besitzt eine Länge e. Die Länge e beschreibt den Abstand zwischen dem vorderen Punkt 13a und dem ersten Scheitelpunkt. Die Länge c beschreibt die Länge des Facettenschliffs 12. Insbesondere beträgt c = 0,5 mm bis 1,5 mm, bevorzugt 0,8 mm bis 1,2 mm. Die Länge b beschreibt den Abstand zwischen dem zweiten Scheitelpunkt 5 und der hinteren äußeren Kante des Grundschliffs 11. Die Strecke g beschreibt die Positionstoleranz des Einstechbereichs 13a um die Mittelachse M des Kanülenrohrs (siehe Figur 3.c). Die Länge der Kanüle 1 ist abhängig von der Höhe des Ports 30 und der Positionstiefe des Ports 30 im Gewebe 40. Die Kanüle kann eine Länge L von L = 3 cm bis 6 cm besitzen.

Um die Herstellung der erfindungsgemäßen Kanüle 1 zu illustrieren, sind in den Figuren 3.a bis 3.c einzelne Verfahrensschritte angedeutet. Zunächst wird das Kanülenrohr 2 in einem geraden Zustand bereitgestellt. Der Anschliff 10 soll am Endabschnitt 4 eingebracht werden. Der Anschliff 10 setzt sich hier aus dem Grundschliff 11 und den beiden Facettenschliffen 12 zusammen. Dazu wird in einem ersten Schritt der Grundschliff 11 unter einem Winkel β eingebracht. Anschließend werden die beiden Facettenschliffe 12 unter einem Winkel γ eingebracht (Figur 3.a). Das Aufrauen der Oberfläche in dem Abschnitt 6 erfolgt vorzugsweise bevor der Anschliff 10 eingebracht wird.

In einem nächsten Schritt wird zunächst die zweite Biegung 5 eingebacht. Die zweite Biegung 5 wird vor der ersten Biegung 3 bereitgestellt. Denn die Benennung der Biegungen 3 und 5 ergibt sich nicht aus der zeitlichen Reihenfolge ihrer Herstellung sondern aus der Reihenfolge, wie die Biegungen 3 und 5, ausgehend von der Vorderseite bzw. dem Einstechbereich 13a der Kanüle 1 aus angeordnet sind. Der Endabschnitt 4 wird über die zweite Biegung 5 um einen Winkel α gebogen (Figur 3.b). Der Endabschnitt 4 wird von der Mittelachse M des Kanülenrohrs 2 weg gebogen.

In einem weiteren Schritt wird die nun erste Biegung 3 eingebracht. Dazu wird die Kanülenspitze 13 in Richtung zur Mittelachse M gebogen. Die Kanülenspitze 13 wird an ihrer Vorderseite durch einen Einstechbereich 13a bereitgestellt. Durch das Biegen oder Krümmen der Kanülenspitze 13 in Richtung der Mittelachse M wird die Hinterkante 14 nun wesentlich verdeckt. Um den Ausstanzeffekt noch weiter zu verringern, wird der Abschnitt 15, welcher die Hinterkante 14 umfasst, noch verrundet.

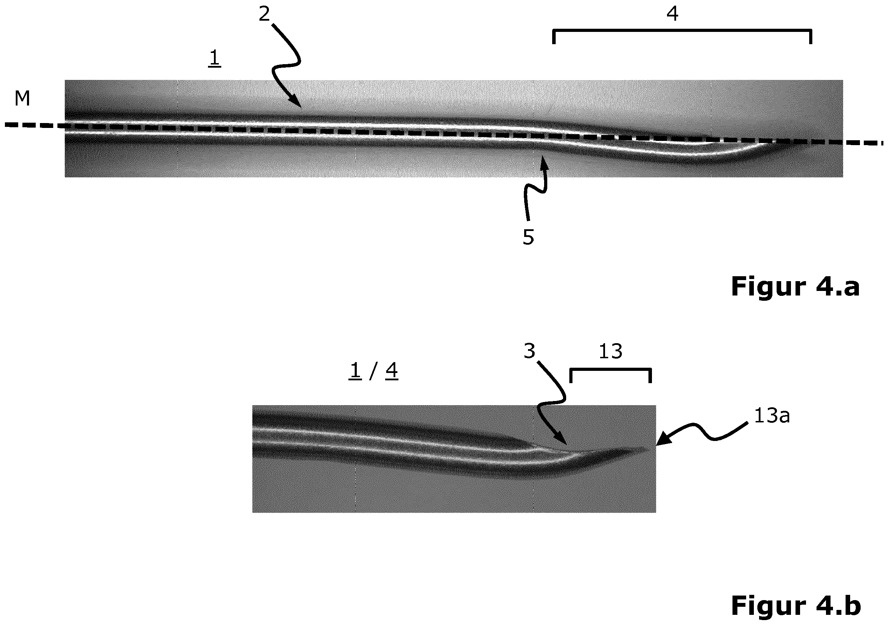

Um den Aufbau der Kanüle 1 noch einmal zu demonstrieren, sind in den Figuren 4.a und 4.b Fotographien einer erfindungsgemäßen Kanüle 1 dargestellt. Deutlich zu erkennen ist zum einen die zweite Biegung 5, mittels derer der Endabschnitt 4 der Kanüle 1 aus der Mittelachse M des Kanülenrohrs 2 herausgebogen ist. Zum anderen ist auch die erste Biegung 3 zu erkennen, mittels derer die Kanülenspitze 13 wieder in Richtung der Mittelachse M der Kanülenrohrs 2 gebogen ist, um den Lumen 2a des Kanülenrohrs 2 zu verdecken.

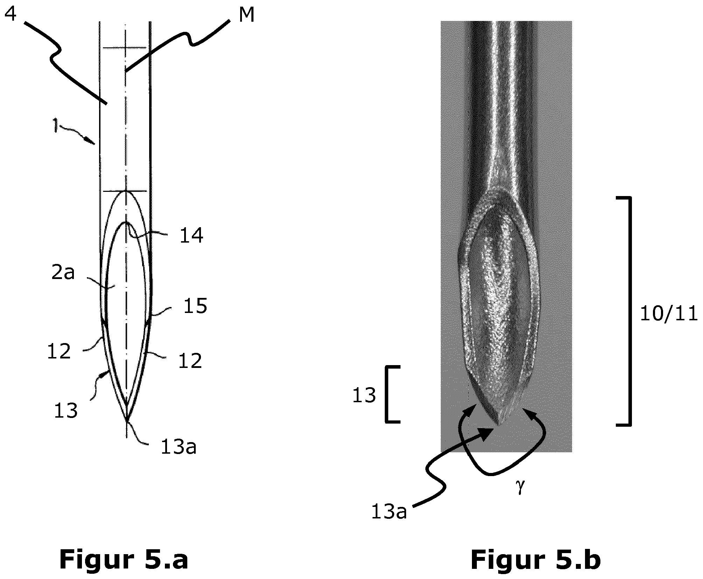

Schließlich zeigen die Figuren 5.a und 5.b zum Vergleich eine schematische Darstellung (Fig. 5.a) und ein Fotografie (Fig. 5.b) einer erfindungsgemäßen Kanüle 1 und ihres Endabschnitts 4. Deutlich zu erkennen sind die, vorzugsweise im kurzen und/oder mittleren Bereich liegende, Schliffausführung des Grundschliffs 11, der verkürzte Facettenschliff 12 und der größeren Winkel γ des Facettenschliffs 12. Durch die erfindungsmäße Kanüle wird insbesondere sowohl ein Ausstanzen von Membranmaterial beim Einführen der Kanüle 1 in den Portkatheter 30 als auch beim Herausziehen der Kanüle 1 aus dem Portkatheter 30 wesentlich reduziert.

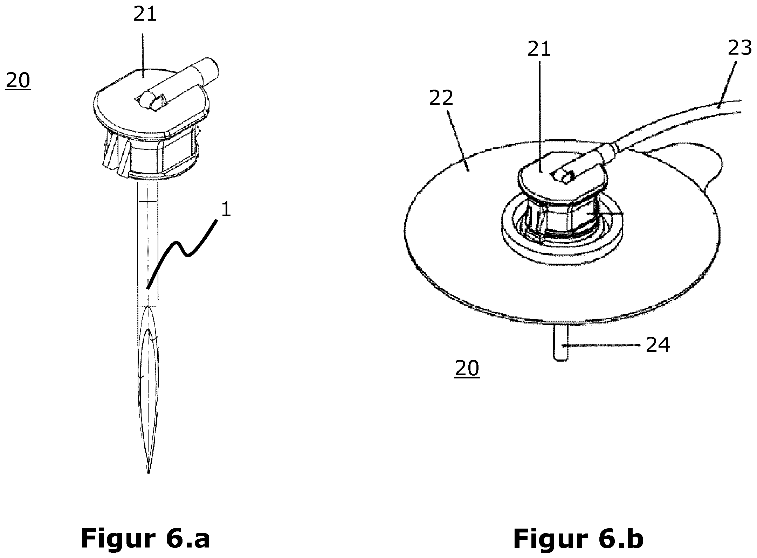

Abschließend zeigen die Figuren 6.a und 6.b eine schematische Ansicht eines Portkanülensystems 20 ohne (Fig. 6.a) und mit Auflage 22 (Fig. 6.a). Die Zuführung 21 ist ein Anschlusskörper zum Verbinden der Kanüle 1 mit einem Schlauch 23, zum Beispiel eines Überleitsystems zu einem nicht dargestellten Infusionsbeutel, der einen zu applizierenden Wirkstoff enthält. Dazu zeugt Figur 6.b eine beispielhafte Ausgestaltung mit einem Pflaster als Auflage 22. Ferner weist das Portkanülensystem 20 noch eine Stichschutzvorrichtung 24, beispielsweise in Form eines Röhrchens 24, auf. Die Kanüle 1 ist in dem Röhrchen angeordnet.

Es ist dem Fachmann ersichtlich, dass die beschriebenen Ausführungsformen beispielhaft zu verstehen sind. Die Erfindung ist nicht auf diese beschränkt sondern kann in vielfältiger Weise variiert werden, ohne das Wesen der Erfindung zu verlassen. Merkmale einzelner Ausführungsformen und die im allgemeinen Teil der Beschreibung genannten Merkmale können jeweils untereinander als auch miteinander kombiniert werden.

### Bezugszeichenliste:

- 1: Kanüle oder Portkanüle oder Nadel
- 2: Kanülenrohr
- 2a: Lumen des Kanülenrohrs
- 3: Erste Biegung oder Krümmung
- 4: Endabschnitt der Kanüle
- 5: Zweite Biegung oder Krümmung
- 6: Abschnitt des Kanülenrohrs mit aufgerauter Oberfläche

- 10: Anschliff
- 11: Grundschliff
- 12: Facettenschliff
- 13: Kanülenspitze
- 13a: vorderer Bereich oder Einstechbereich oder Einstechpunkt der Kanülenspitze
- 14: Hinterkante des Grundschliffs
- 15: Bereich des Grundschliffs zwischen den Facettenschliffen

- 20: Portkanülensystem
- 21: Zuführung oder Anschlusskörper
- 22: Auflage oder Pflaster
- 23: Schlauch

- 30: Port oder Portsystem oder Portkatheter
- 31: Portgehäuse oder Gehäuse
- 32: Portmembran oder Membran
- 33: Anschlussstück für einen Katheter
- 34: Katheter
- 35: Befestigungsmittel für den Port

- 40: Gewebe
- 41: Haut
- M: Mittelachse

- a: Länge des Anschliffbereichs oder des Grundschliffs
- b: Abstand zwischen dem Grundschliffansatz und dem zweitem Scheitelpunkt der zweiten Biegung
- c: Länge des Facettenschliffs
- d: Außendurchmesser der Kanüle
- e: Abstand zwischen dem Einstechpunkt der Kanüle und dem ersten Scheitelpunkt der ersten Biegung
- f: Länge des Endabschnitts oder Abstand zwischen dem Einstechpunkt der Kanüle und dem zweitem Scheitelpunkt der zweiten Biegung (f = a + b)
- g: Abstand des Einstechbereichs von der Mittelachse
- r: Biegeradius

- α: Neigungswinkel der zweiten Biegung
- β: Grundschliffwinkel
- γ: Winkel zwischen den Facettenschliffen

- M: Mittelachse

## Patentansprüche

1. Kanüle (1) zum Punktieren eines implantierbaren Ports (30) wenigstens aus
einem Kanülenrohr (2) mit einem Anschliff (10), welcher sich zumindest aus einem gegenüber einer Mittelachse (M) des Kanülenrohrs (2) um einen Grundschliffwinkel (β) geneigten Grundschliff (11) und zwei im Winkel (γ) zueinander stehenden Facettenschliffen (12) zusammensetzt, und einer durch den Anschliff (10) bereitgestellten Kanülenspitze (13), welche über eine erste Biegung (3) in Richtung auf die Mittelachse (M) der Kanülenrohrs (2) gerichtet ist, **dadurch gekennzeichnet, dass**,
der Grundschliffwinkel (β) in einem Bereich von β = 13° bis 22° liegt und der Winkel (γ) zwischen den zwei Facettenschliffen (12) in einem Bereich von γ = 90° bis 120° liegt.

2. Kanüle (1) nach vorstehendem Anspruch, **dadurch gekennzeichnet, dass** der Grundschliff (11) einen Anschliffbereich der Länge a aufweist und wenigstens einer der zwei Facettenschliffe (12) eine Länge c von c < 1/3 a aufweist.

3. Kanüle (1) nach einem der vorstehenden Ansprüche, **dadurch gekennzeichnet, dass** ein Lumen (2a) des Kanülenrohrs (2) durch die Kanülenspitze (13) zumindest abschnittweise verdeckt ist.

4. Kanüle (1) nach einem der vorstehenden Ansprüche, **dadurch gekennzeichnet, dass** eine durch den Grundschliff (11) bereitgestellte Hinterkante (14) durch die Kanülenspitze (13) zumindest abschnittsweise verdeckt ist.

5. Kanüle (1) nach einem der vorstehenden Ansprüche, **dadurch gekennzeichnet, dass** ein Einstechbereich (13a) der Kanülenspitze (13) in einem Bereich g von 0,5 mm oberhalb und 0,2 mm unterhalb, bevorzugt 0,2 mm oberhalb und 0,1 mm unterhalb, der Mittelache (M) des Kanülenrohrs (2) liegt.

6. Kanüle (1) nach einem der vorstehenden Ansprüche, **dadurch gekennzeichnet, dass** die erste Biegung (3) der Kanülenspitze (13) durch einen, vorzugsweise einzelnen, ersten Biegeradius (r) bereitgestellt ist, welcher insbesondere in einem Bereich von r = 5 mm bis 10 mm, bevorzugt von 7 mm bis 9 mm, liegt.

7. Kanüle (1) nach einem der vorstehenden Ansprüche, **dadurch gekennzeichnet, dass** die erste Biegung (3) der Kanülenspitze (13) einen ersten Scheitelpunkt aufweist, der in einem Abstand e = 3 mm bis 5 mm, bevorzugt von 3,6 mm bis 4 mm zu einem Einstechbereich (13a) der Kanülenspitze (13) liegt.

8. Kanüle (1) nach einem der vorstehenden Ansprüche, **dadurch gekennzeichnet, dass** ein zwischen den zwei Facettenschliffen (12) liegender Abschnitt (15) des Grundschliffs (11) zumindest abschnittsweise, vorzugsweise vollständig, verrundet ist.

9. Kanüle (1) nach einem der vorstehenden Ansprüche, **dadurch gekennzeichnet, dass** ein Endabschnitt (4) der Kanüle (1), an dem der Anschliff (10) angeordnet ist, über eine zweite Biegung (5) von der Mittelachse (M) der Kanülenrohrs (2) weg gerichtet ist.

10. Kanüle (1) nach vorstehendem Anspruch, **dadurch gekennzeichnet, dass** der Endabschnitt (4) der Kanüle (1), an dem der Anschliff (10) angeordnet ist, über die zweite Biegung (5) in eine Richtung gebogen ist, welche der Richtung der ersten Biegung (3) gegenüberliegt.

11. Kanüle (1) nach einem der beiden vorstehenden Ansprüche, **dadurch gekennzeichnet, dass** die zweite Biegung (5) des Endabschnitts (4) einen zweiten Scheitelpunkt aufweist, der in einem Abstand f = 4 mm bis 10 mm, bevorzugt f = 6 mm bis 10 mm, besonders bevorzugt f = 6 mm bis 8 mm, zu einem Einstechbereich (13a) der Kanülenspitze (13) liegt.

12. Kanüle (1) nach einem der vorstehenden Ansprüche, **dadurch gekennzeichnet, dass**
der Endabschnitt (4) der Kanüle (1), an dem der Anschliff (10) angeordnet ist, in einem spitzen Winkel (α) zu der Mittelachse (M) des Kanülenrohrs (2) angeordnet ist und
insbesondere der spitze Winkel (α) in meinem Bereich von α = 1 bis 10°, bevorzugt α = 4° bis 7°, liegt.

13. Kanüle (1) nach einem der vorstehenden Ansprüche, **dadurch gekennzeichnet, dass** zumindest ein Abschnitt (6) einer Außenseite des Kanülenrohrs (2) eine aufgeraute Oberfläche hat, der sich vorzugsweise nicht vollständig über einen Umfang des Kanülenrohrs (2) erstreckt und insbesondere eine Rauigkeit Rz aufweist, die in einem Bereich von Rz = 1 µm bis 10 µm, bevorzugt von Rz = 5 µm bis 10 µm, liegt.

14. Portkanülensystem (20) umfassend ein Kanüle (1) nach einem der vorstehenden Ansprüche und eine mit der Kanüle (1) verbundene Zuführung (21) zum Anschließen eines Schlauchs (23) eines Überleitsystems, die an einem dem Endabschnitt (4) gegenüberliegenden Abschnitt des Kanülenrohrs (2) angeordnet ist sowie insbesondere einer mit der Zuführung (21) verbundenen Auflage (22) zur Auflage des Portkanülensystems (20) auf der Haut (41) eines Patienten.

15. Portkanülensystem (20) nach vorstehendem Anspruch, **dadurch gekennzeichnet, dass** die Zuführung (21) an dem Abschnitt (6) der Außenseite des Kanülenrohrs (2) mit der aufgerauten Oberfläche angeordnet ist.

## Claims

1. Needle (1) for the puncturing of an implantable port (30), consisting at least of
a needle tube (2) with a ground surface (10), which is formed at least from a ground cut (11) inclined with respect to a center axis (M) of the needle tube (2) by a ground-cut angle (β) and from two facet cuts (12) lying at an angle (γ) to each other, and of a needle tip (13) which is provided by the ground surface (10) and which is oriented via a first bend (3) in the direction of the center axis (M) of the needle tube (2), **characterized in that**
the ground-cut angle (β) lies in a range of β = 13° to 22°, and the angle (γ) between the two facet cuts (12) lies in a range of γ = 90° to 120°.

2. Needle (1) according to the preceding claim, **characterized in that** the ground cut (11) has a ground-surface area of length a, and at least one of the two facet cuts (12) has a length c of c < 1/3 a.

3. Needle (1) according to one of the preceding claims, **characterized in that** a lumen (2a) of the needle tube (2) is at least partially concealed by the needle tip (13).

4. Needle (1) according to one of the preceding claims, **characterized in that** a rear edge (14) provided by the ground cut (11) is at least partially concealed by the needle tip (13).

5. Needle (1) according to one of the preceding claims, **characterized in that** a piercing area (13a) of the needle tip (13) lies in a range g of 0.5 mm above and 0.2 mm below, preferably 0.2 mm above and 0.1 mm below, the center axis (M) of the needle tube (2).

6. Needle (1) according to one of the preceding claims, **characterized in that** the first bend (3) of the needle tip (13) is provided by a preferably single first bend radius (r), which lies in particular in a range of r = 5 mm to 10 mm, preferably of 7 mm to 9 mm.

7. Needle (1) according to one of the preceding claims, **characterized in that** the first bend (3) of the needle tip (13) has a first vertex, which lies at a distance e = 3 mm to 5 mm, preferably 3.6 mm to 4 mm, from a piercing area (13a) of the needle tip (13).

8. Needle (1) according to one of the preceding claims, **characterized in that** a portion (15) of the ground cut (11) lying between the two facet cuts (12) is at least partially rounded, preferably completely rounded.

9. Needle (1) according to one of the preceding claims, **characterized in that** an end portion (4) of the needle (1), at which the ground surface (10) is arranged, is oriented away from the center axis (M) of the needle tube (2) via a second bend (5).

10. Needle (1) according to the preceding claim, **characterized in that** the end portion (4) of the needle (1), at which the ground surface (10) is arranged, is curved, via the second bend (5), in a direction that is opposite to the direction of the first bend (3).

11. Needle (1) according to one of the two preceding claims, **characterized in that** the second bend (5) of the end portion (4) has a second vertex, which lies at a distance f = 4 mm to 10 mm, preferably f = 6 mm to 10 mm, particularly preferably f = 6 mm to 8 mm, from a piercing area (13a) of the needle tip (13).

12. Needle (1) according to one of the preceding claims, **characterized in that**
the end portion (4) of the needle (1), at which the ground surface (10) is arranged, is arranged at an acute angle (α) to the center axis (M) of the needle tube (2), and
in particular, the acute angle (α) lies in a range of α = 1° to 10°, preferably α = 4° to 7°.

13. Needle (1) according to one of the preceding claims, **characterized in that** at least a portion (6) of an outside of the needle tube (2) has a roughened surface, which preferably does not extend completely about a circumference of the needle tube (2) and in particular has a roughness Rz that lies in a range of Rz = 1 µm to 10 µm, preferably of Rz = 5 µm to 10 µm.

14. Port needle system (20) comprising a needle (1), according to one of the preceding claims, and a feeder (21) which is connected to the needle (1) and permits attachment of a hose (23) of a transfer system, which feeder (21) is arranged on a portion of the needle tube (2) opposite the end portion (4), and in particular a support piece (22) which is connected to the feeder (21) and is designed for placing the port needle system (20) on the skin (41) of a patient.

15. Port needle system (20) according to the preceding claim, **characterized in that** the feeder (21) is arranged on the portion (6) of the outside of the needle tube (2) having the roughened surface.

## Revendications

1. Canule (1) destinée au pointage d'une chambre implantable (30), composée au moins d'un tube de canule (2) avec une section polie (10), qui se compose au moins d'une section polie de base (11) inclinée d'un angle de section polie de base (β) par rapport à un axe central (M) du tube de canule (2) et de deux facettes polies (12) formant un angle (γ) l'une avec l'autre, et d'une pointe de canule (13) formée par la section polie (10), qui est dirigée au moyen d'une première courbure (3) en direction de l'axe central (M) du tube de canule (2), **caractérisée en ce que** l'angle de section polie de base (β) se situe dans une plage de β = 13° à 22° et l'angle (γ) entre les deux facettes polies (12) se situe dans une plage de γ = 90° à 120°.

2. Canule (1) selon la revendication précédente, **caractérisée en ce que** la section polie de base (11) présente une zone de section polie de longueur a et au moins une des deux facettes polies (12) présente une longueur c avec c < 1/3 a.

3. Canule (1) selon l'une quelconque des revendications précédentes, **caractérisée en ce qu'**une lumière (2a) du tube de canule (2) est couverte au moins en partie par la pointe de canule (13).

4. Canule (1) selon l'une quelconque des revendications précédentes, **caractérisée en ce qu'**une arête arrière (14) de la section polie de base (11) est couverte au moins en partie par la pointe de canule (13).

5. Canule (1) selon l'une quelconque des revendications précédentes, **caractérisée en ce qu'**une zone de pénétration (13a) de la pointe de canule (13) se situe dans une plage g de 0,5 mm au-dessus à 0,2 mm en dessous, de préférence de 0,2 mm au-dessus à 0,1 mm en dessous de l'axe central (M) du tube de canule (2).

6. Canule (1) selon l'une quelconque des revendications précédentes, **caractérisée en ce que** la première courbure (3) de la pointe de canule (13) est formée avec un premier, de préférence un seul, rayon de courbure (r), qui se situe en particulier dans une plage de r = 5 mm à 10 mm, de préférence de 7 mm à 9 mm.

7. Canule (1) selon l'une quelconque des revendications précédentes, **caractérisée en ce que** la première courbure (3) de la pointe de canule (13) présente une première clé de voûte, qui se situe à une distance e = 3 mm à 5 mm, de préférence de 3,6 mm à 4 mm d'une zone de pénétration (13a) de la pointe de canule (13).

8. Canule (1) selon l'une quelconque des revendications précédentes, **caractérisée en ce qu'**une partie (15) de la section polie de base (11) située entre les deux facettes polies (12) est au moins en partie, de préférence totalement, arrondie.

9. Canule (1) selon l'une quelconque des revendications précédentes, **caractérisée en ce qu'**une partie d'extrémité (4) de la canule (1), sur laquelle la section polie (10) est située, est écartée de l'axe central (M) du tube de canule (2) au moyen d'une deuxième courbure (5).

10. Canule (1) selon la revendication précédente, **caractérisée en ce que** la partie d'extrémité (4) de la canule (1), sur laquelle la section polie (10) est située, est courbée au moyen de la deuxième courbure (5) dans une direction qui est opposée à la direction de la première courbure (3).

11. Canule (1) selon l'une quelconque des deux revendications précédentes, **caractérisée en ce que** la deuxième courbure (5) de la partie d'extrémité (4) présente une deuxième clé de voûte, qui se situe à une distance f = 4 mm à 10 mm, de préférence f = 6 mm à 10 mm, et de préférence encore f = 6 mm à 8 mm, d'une zone de pénétration (13a) de la pointe de canule (13).

12. Canule (1) selon l'une quelconque des revendications précédentes, **caractérisée en ce que** la partie d'extrémité (4) de la canule (1), sur laquelle la section polie (10) est située, est disposée sous un angle aigu (α) par rapport à l'axe central (M) du tube de canule (2), et l'angle aigu (α) se situe en particulier dans une plage de α = 1° à 10°, de préférence α = 4° à 7°.

13. Canule (1) selon l'une quelconque des revendications précédentes, **caractérisée en ce qu'**au moins une partie (6) d'un côté extérieur du tube de canule (2) présente une surface dépolie, qui ne s'étend de préférence pas entièrement sur une périphérie du tube de canule (2) et présente en particulier une rugosité Rz, qui se situe dans une plage de Rz = 1 µm à 10 µm, de préférence de Rz = 5 µm à 10 µm.

14. Système de canule à chambre (20) comprenant une canule (1) selon l'une quelconque des revendications précédentes et un dispositif d'alimentation (21) raccordé à la canule (1) pour le raccordement d'un tuyau flexible (23) d'un système de transfert, qui est disposé sur une partie du tube de canule (2) située à l'opposé de la partie d'extrémité (4), et en particulier un appui (22) raccordé au dispositif d'alimentation (21) pour le dépôt du système de canule à chambre (20) sur la peau (41) d'un patient.

15. Système de canule à chambre (20) selon la revendication précédente, **caractérisé en ce que** le dispositif d'alimentation (21) est disposé sur la partie (6) du côté extérieur du tube de canule (2) dotée de la surface dépolie.
